# EUROPEAN PATENT SPECIFICATION

(11) **EP 3 155 663 B1**
(45) Date of publication and mention of the grant of the patent: **16.05.2018**
(21) Application number: 15730278.7
(22) Date of filing: 19.05.2015
(51) Int. Cl.: H01L 27/146, G01T 1/20

(54) **MODULAR IMAGING DETECTOR ASIC**
MODULARER BILDGEBUNGSDETEKTOR-ASIC
CIRCUIT ASIC DE DÉTECTEUR D'IMAGERIE MODULAIRE

(30) Priority: 10.06.2014 US 201462009974 P
(43) Date of publication of application: 19.04.2017
(73) Proprietor: Koninklijke Philips N.V., 5656 AE Eindhoven (NL)
(72) Inventor: CHAPPO, Marc Anthony, NL-5656 AE Eindhoven (NL); GOSHEN, Rafael, NL-5656 AE Eindhoven (NL)
(74) Representative: Versteeg, Dennis John
(86) International application number: PCT/IB2015/053665
(87) International publication number: WO 2015/189728

(56) References cited:
- WO-A1-2013/164717
- LUTHA R ET AL: "A new 2D-tiled detector for multislice CT", PROGRESS IN BIOMEDICAL OPTICS AND IMAGING, SPIE - INTERNATIONAL SOCIETY FOR OPTICAL ENGINEERING, BELLINGHAM, WA, US, vol. 6142, 12 February 2006 (2006-02-12), pages 61420U-1, XP002583785, ISSN: 1605-7422, DOI: 10.1117/12.653245 ISBN: 978-1-62841-502-5 [retrieved on 2006-03-02]

## Description

The following generally relates to imaging and more particularly to an imaging detector that includes at least a sub-portion of a modular imaging detector application specific integrated circuit (ASIC), and is described with particular application to computed tomography (CT). However, the following is also amenable to other imaging modalities.

A computed tomography (CT) scanner includes an x-ray tube mounted on a rotatable gantry that rotates around an examination region about a longitudinal or z-axis. The x-ray tube emits ionizing radiation that traverses the examination region and a subject or object therein. A detector array subtends an angular arc opposite the examination region from the x-ray tube. The detector array detects radiation that traverses the examination region and generates a signal indicative thereof. A reconstructor processes the signal and reconstructs volumetric image data indicative of the examination region. The reconstructed volumetric image can be further processed to generate one or more images of the examination region. Document WO2013/164717 shows a tiled x-ray imaging detector. The detector array has included an integrating detector with a scintillator array optically coupled and mounted to one side of a photosensor array and an ASIC mounted to an opposing side of the photosensor array. The ASIC includes channels that are in electrical communication with electrical contacts to the photosensitive pixels. For example, a detector with a photosensor array with 64 photosensitive pixels has an ASIC with 64 corresponding channels. Generally, the ASIC is optimized for the specific numbers of pixels with, for example, 64, 128, 256 or 512 channels. However, this limits the applicability of an ASIC to a particular product line. For example, an ASIC optimized for 64 channels, generally, is limited to an imaging system with a detector array with a photosensor array with 64 photosensor pixels.

As a consequence, multiple different detector configurations (e.g., 64, 128, 256, 512, etc. photosensitive pixels) for different imaging system configurations require corresponding multiple different optimized ASICs (e.g., 64, 128, 256, 512, etc. channels). Unfortunately, this leads to manufacturing inefficiencies. For example, development of a specific ASIC may take two 2 years with each costing in excess of one million dollars, and each ASIC is characterized and qualified with special test equipment. In another example, an ASIC (which is optimized for a 128 photosensitive pixel detector) that has improperly functioning channels at testing would be discarded, lowering the overall yield. An approach for overcoming some of the inefficiencies is to just use higher end ASICs (e.g., 512 channels) for all imaging system configurations. Unfortunately, this may be size and cost prohibitive for lower (e.g., less than 512 photosensor pixel) end imaging systems.

Aspects described herein address the above-referenced problems and others.

The following describes a single modular ASIC, which is divisible into one or more fully functional reduced channel ASICs that can be used in different detector configurations (e.g., 1, 2, 4, 8, 16, 32, 64, 128, 256, 512, 1024, etc. pixels). With this approach, an ASIC with a larger number of channels (e.g. 512 or more channels) can be fabricated (e.g., with a small feature size of 0.05 micron to 0.20 micron) and tested, and then used in a high definition detector or physically divided to produce one or more reduced channel ASICs (e.g., 64 channel) for a lower definition or lower pixel count detector. As a result, ASICs with a different number of channels can be produced from a single fabrication, optimizing both cost and size across the different detector configurations.

In one aspect, an imaging system detector array includes a detector tile. The detector tile includes a photosensor array, including a plurality of photosensor pixels. The detector tile further includes a scintillator array optically coupled to the photosensor array. The detector tile further includes an ASIC electronics layer that is electrically coupled to the photosensor array. The electronics layer includes a plurality of individual processing regions. Each processing region includes a predetermined number of channels corresponding to a sub-set of the plurality of photosensor pixels. The processing regions are in electrical communication with each other. Each processing region includes its own electrical reference and bias circuitry.

In another aspect, a method includes fabricating and testing an ASIC that includes a first number of processing regions. Each processing region includes a predetermined number of channels. Each processing region includes its own electrical reference and bias circuitry. The method further includes dicing the ASIC into at least two fully functional reduced channel ASICS respectively having a second and a third number of channels. The second and the third number of channels is less that the first number of channels. The method further includes employing at least one of the at least two fully functional reduced channel ASICS in a detector array of an imaging system.

In another aspect, an ASIC for an imaging detector tile includes a plurality of individual processing regions, each processing region including a predetermined number of channels corresponding to a sub-set of a plurality of photosensor pixels of the detector tile, wherein the processing regions are in electrical communication with each other, and each processing region includes its own electrical reference and bias circuitry.

The invention may take form in various components and arrangements of components, and in various steps and arrangements of steps. The drawings are only for purposes of illustrating the preferred embodiments and are not to be construed as limiting the invention.
FIGURE 1 schematically illustrates an example imaging system with a detector array that includes at least a sub-portion of a single modular ASIC that is divisible into one or more fully functional reduced channel ASICs.
FIGURE 2 schematically illustrates an example detector tile that includes the single modular ASIC.
FIGURE 3 schematically illustrates an example of the single modular ASIC with MxN processing regions.
FIGURE 4 schematically illustrates an example of the single modular ASIC with four (M=N=2, or 2x2) processing regions and digital and analog regions.
FIGURE 5 schematically illustrates an example of dicing a reduced channel ASIC from the single modular ASIC of FIGURE 4.
FIGURE 6 schematically illustrates another example of dicing a reduced channel ASIC from the single modular ASIC of FIGURE 4.
FIGURE 7 schematically illustrates still another example of dicing a reduced channel ASIC from the single modular ASIC of FIGURE 4.
FIGURE 8 schematically illustrates an example of the ASIC in which each processing region includes its own internal reference and bias circuitry.
FIGURE 9 schematically illustrates the ASIC in FIGURE 8 in which the ASIC is diced.
FIGURE 10 schematically illustrates a side view of the ASIC, showing metal layers of the ASIC.
FIGURE 11 schematically illustrate a top down view of the ASIC, showing electrical connections across processing regions of the ASIC.
FIGURE 12 schematically illustrates a variation of FIGURE 4 in which the processing regions are located between the digital regions.
FIGURE 13 schematically illustrates a variation of FIGURE 8 in which the reference and bias circuitry is external to the processing regions.
FIGURE 14 illustrates a method in accordance with the system described herein.

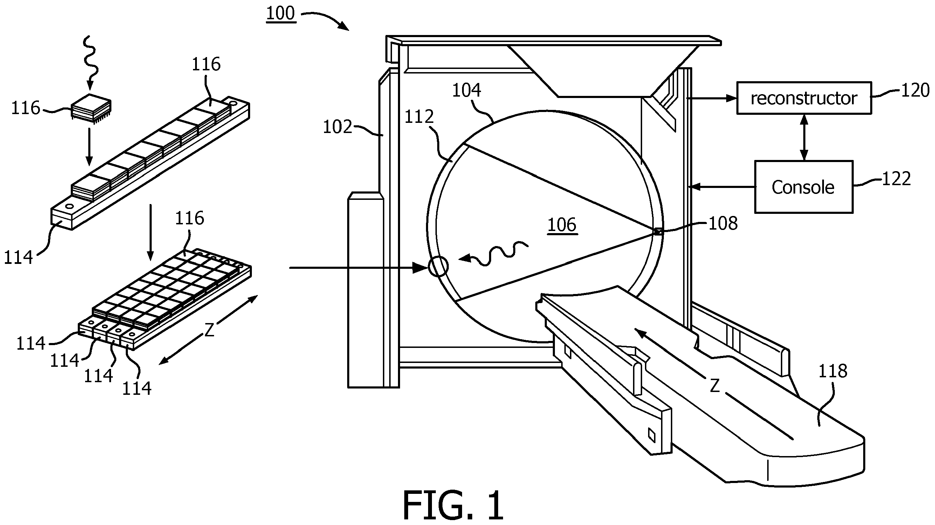

FIGURE 1 illustrates an imaging system 100 such as a computed tomography (CT) scanner. The imaging system 100 includes a generally stationary gantry 102 and a rotating gantry 104. The rotating gantry 104 is rotatably supported by the stationary gantry 102 and rotates around an examination region 106 about a longitudinal or z-axis. A radiation source 108 such as an x-ray tube is supported by and rotates with the rotating gantry 104 and emits radiation that traverses the examination region 106.

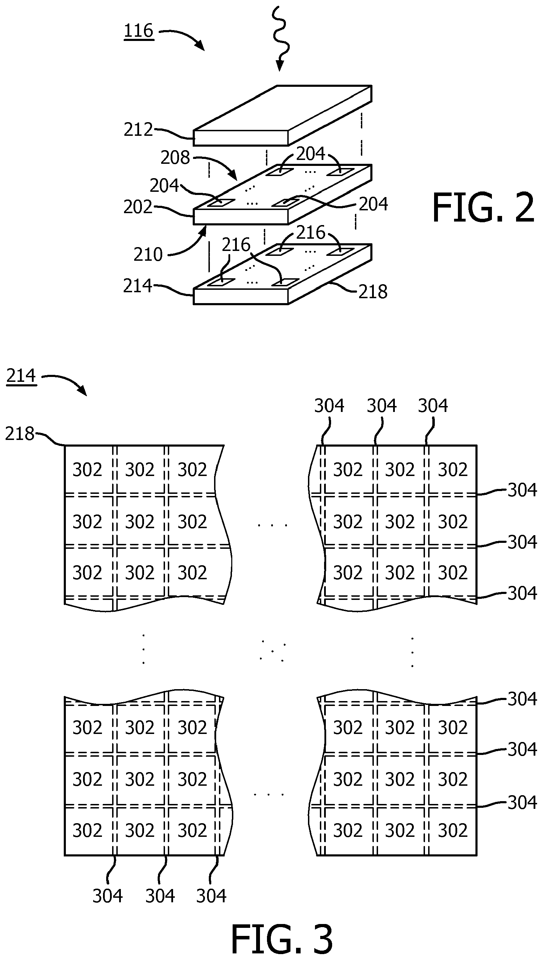

A radiation sensitive detector array 112 subtends an angular arc opposite the radiation source(s) 108 across the examination region 106 and detects radiation traversing the examination region 106. The radiation sensitive detector array 112 includes a plurality of detector modules 114 arranged with respect to each other along a direction transverse to the z-axis. A detector module 114 includes a plurality of detector mosaics or tiles 116 arranged with respect to each other along the z-axis. A non-limiting example of such a detector array is described in US patent 6,510,195B1, filed July 18, 2001, and entitled "Solid State X-Radiation Detector Modules and Mosaics thereof, and an Imaging Method and Apparatus Employing the Same". Briefly turning to FIGURE 2, an example of a detector tile 116 is schematically illustrated. The detector tile 116 is an integrating detector, including a photosensor layer 202, which includes a plurality of photosensitive pixels 204 on a first side 208. The illustrated photosensor layer 202 is back-illuminated with electrodes (not visible) that inter-connect the photosensitive pixels 204 to bonding pads or the like (not visible) located on a second opposing side 210 of the photosensor 202. In a variation, the photosensor 202 can be a front-illuminated photosensor with vias (including but not limited to through silicon vias, TSVs) that route the signals from the first side 208 to the pads on the opposing side 210. In another variation, the detector tile 116 can be a multi-layer, spectral (multi-energy) detector tile with multiple scintillator/photosensor pairs, each of which is sensitive to a different photon energy range.

The detector tile 116 further includes a scintillator layer 212. The scintillator layer 212 may be a single layer or include a plurality of scintillator pixels (pixelated). In the latter instance, the scintillator layer 212 may include a number of scintillator pixels corresponding to a number of photosensitive areas for a one to one relationship between scintillator pixel and photosensitive area 204. In yet another instance, different scintillator pixels may correspond to different sub-groups of the photosensitive areas 204. The scintillator layer 212 is optically coupled to the photosensor 202.

The detector tile 116 further includes an electronics layer (e.g., an ASIC) 214 with electrically conductive pads 216 that couple with the bonding pads of the photosensitive pixels 204. The ASIC or ASIC on a substrate 214 includes a channel for each of the photosensitive pixels 204, along with digital and analog circuitry. Such circuitry includes an A/D converter, which is implemented as a current-to-frequency (I/F) converter that generates a train of pulses with a pulse frequency indicative of x-ray photons incident on a detector pixel. Examples of such a converter are described in US patent 6,671,345 B2 Vrettos et al., filed November 7, 2001, and entitled "Data Acquisition for Computed Tomography," and "A New 2D-Tiled Detector for Multislice CT," Luhta et al., Medical Imaging 2006: Physics of Medical Imaging, Vol. 6142, pp. 275-286 (2006).

The illustrated ASIC or ASIC on a substrate 214 is a modular ASIC, with a single unitary die 218 that is divisible into one or more reduced channel ASICs that can be used in different detector configurations (e.g., 1, 2, 8, 16, 32, 64, 128, 256, 512, 1024, etc. photosensitive pixels). The single unitary die 218 may include at least one of a flex, a ceramic, a silicon, etc. material, a print circuit board (PCB), etc. As described in greater detail below, the single unitary die 218 is populated with electronics such that the individual reduced-channel ASICs include suitable electronics to be fully functional reduced channel ASICs. As such, manufacturing inefficiencies can be mitigated by fabricating and testing a single modular ASIC 218, and producing desired channel size ASICs 218 therefrom, without introducing size and cost prohibitive constraints on lower end systems.

This approach may also improve yields as an ASIC 218 that has improperly functioning channels can be diced to produce a reduced channel ASIC with properly functioning channels, instead of discarding the entire ASIC or ASIC on a substrate 214. Generally, the number of ASIC channels should fit the number of detection channels so each photodiode will be connected to one preamplifier. If the ASIC has more channels, it will take more power consumption and physical space in an area, which is often already densely populated. In one instance, a particular size ASIC (e.g., 512 channel) is fabricated and qualified, and subsequently used to produce 512 channel or sub units like 256, 128 or 64 channel ASICs. This may result in a direct cost saving and in non-direct cost savings, for example, due to large use of one type of ASIC. The base ASIC will serve many types of detectors, each one with different number of input channels.

Returning to FIGURE 1, a patient support 118, such as a couch, supports an object or subject such as a human patient in the examination region 106. The patient support 118 is configured to move the object or subject in and/or out of the examination region 106 before, during and/or after scanning the object or subject. A reconstructor 120 reconstructs the signal from the detector array 112 and generates volumetric image data indicative thereof. The volumetric image data can be further processed to generate one or more images, which can be presented via a display, filmed, or otherwise output.

A general-purpose computing system serves as an operator console 122. The console 122 includes one or more processors that execute one or more computer readable instructions (software) stored or encoded in computer readable storage medium local or remote to the system 100. Software resident on the console 122 allows the operator to control operation of the system 100 initiating scanning, etc. The console 122 also includes an output device such as display and an input device such as a keyboard, mouse, touch screen, etc.

FIGURE 3 schematically illustrates an example of the single modular ASIC or ASIC on a substrate 214. The ASIC 218 includes individually and independently fully functioning processing regions 302. Each processing region 302 includes channels that interface with the photosensitive pixels 204 (FIGURE 2) and circuitry for processing electrical signals generated by and obtained from the photosensitive pixels 204.

In the illustrated embodiment, the processing regions 302 are separated from each other by dividing regions 304 (which are delineated in FIGURE 3 through imaginary dashed lines) that reside within common or shared regions of the ASIC, for example, shared analog and/or digital regions shared across processing regions 302. In FIGURE 3, there are N rows and M columns of processing regions 302, where N and M are positive integers

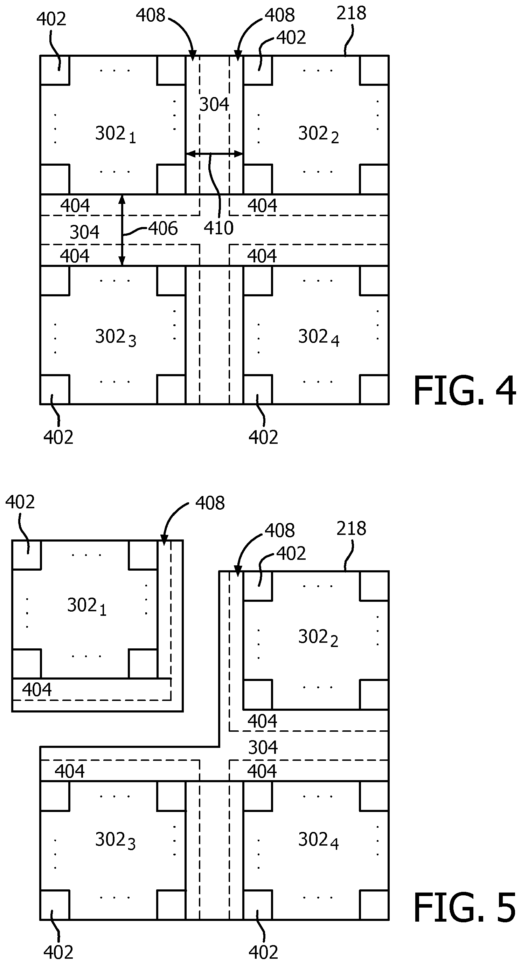

FIGURE 4 shows the ASIC 218 of FIGURE 3 for N = M = 2. In FIGURE 4, each of the processing regions 302₁, 302₂, 302₃, and 302₄ includes an array of J channels 402 (where J is a positive integer) and a sub-portion 404 of a common digital region 406 and a sub-portion 408 of a common analog region 410. The dividing regions 304 are within the common digital region 406 and the common analog region 410.

Turning to FIGURE 5, one of the processing regions 302 (i.e., processing region 302₁) is shown diced and separated from the single modular ASIC 214. The ASIC 218 die is diced to those skilled in the art of ASIC fabrication, for example, by laser cutting, mechanical sawing, scribing and breaking, and/or other dicing. A small percentage of the ASIC die will be lost due to the dicing, for example, about 150 microns or less. However, the ASIC 218 can be fabricated to take this into account, if needed.

In FIGURE 5, the ASIC 218 is diced within the dividing regions 304. The single modular ASIC 218, prior to dicing, included four processing regions 302, each with J channels, providing 4J channels for a 4J photosensor pixel array. The diced processing region 302₁ provides J channels for a J pixel photosensor pixel array. The remainder of the single modular ASIC 218 can be used with a 3J pixel photosensor pixel array or further diced to provide three more J channel ASICs or another J channel ASIC and a 2J channel ASIC.

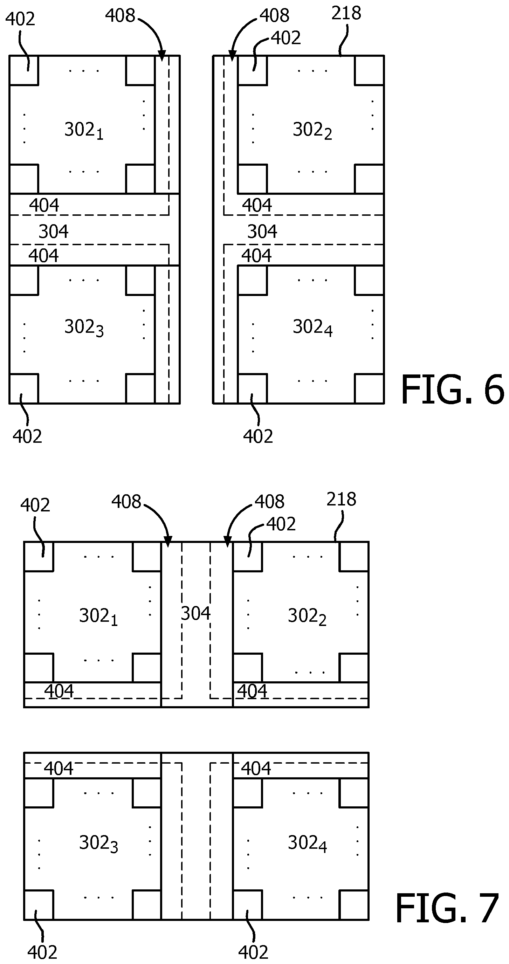

In FIGURE 6, a pair of adjacent processing regions 302 is shown diced from the single modular ASIC 218. In FIGURE 6, the ASIC 218 is diced within the dividing region 304 within the common analog circuitry 408 and results in 2J channels for a 2J photosensor pixel array. The remainder of the single modular ASIC 214 can be used with a 2J photosensor pixel array or further diced to provide two J channel ASICS.

In FIGURE 7, a different pair of adjacent processing regions 302 is shown diced from the single modular ASIC 218. In FIGURE 7, the ASIC 218 is diced within the dividing region 304 within the common digital circuitry 404 and results in 2J channels for a 2J photosensor pixel array. The remainder of the single modular ASIC 218 can be used with a 2J photosensor pixel array or further diced to provide two J channel ASICS.

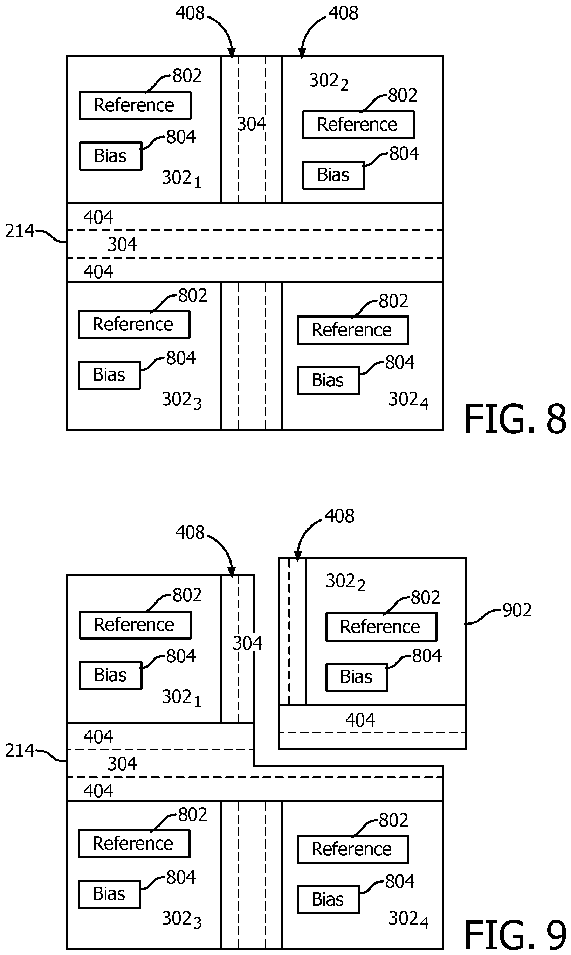

FIGURE 8 schematically illustrates an embodiment of the ASIC 218 in which each of the divisible processing regions 302 includes its own reference circuitry 802 and bias circuitry 804, which in internal to the processing regions 302. As such, the ASIC 218 will include duplicate reference circuitry 802 and bias circuitry 804. With this configuration, dicing the ASIC die, as shown in FIGURE 5 (or otherwise) renders a fully functional reduced channel ASICs 902, as shown in FIGURE 9.

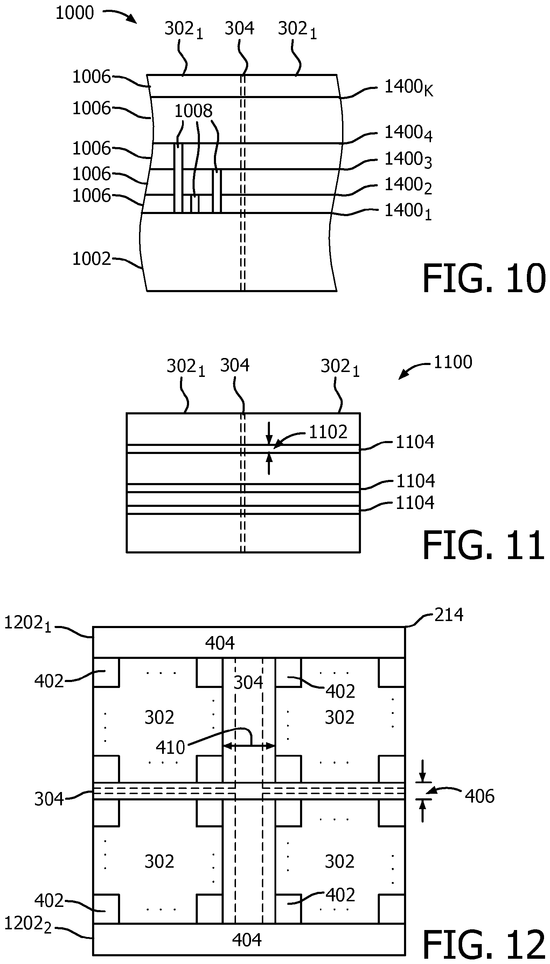

FIGURES 10 and 11 respectively show side and top down views 1200 and 1300 of two processing regions 302₁ and 302₂ of the ASIC 218. The ASIC 218 includes a die substrate 1202 and a plurality of metallization layers 1004₁, 1004₂, 1004₃, 1004₄, ..., 1004ₖ (where k is a positive integer), collectively referred to as of metallization layers 1004. Passivation layers 1006 are between the metallization layers.

Vias 1008 provides paths for electrical connections between the metallization layers 1004 and the substrate 1002. Electrical paths 1004 cross the dividing regions 304. Generally, the separation between the metallization layers 1004 and widths 1102 of electrical paths 1104 that cross between the two processing regions 302₁ and 320₂ are appropriately sized so that the dicing does not render the electrical connections at the edges non-functional.

FIGURE 12 shows a variation of the ASIC 218 of FIGURE 4 in which the processing regions 302 are located between (i.e., sandwiched by) first and second sub-portions 1202₁ and 1202₂ of the digital region 406, instead of the digital circuitry 406 being between (i.e., sandwich by) the processing regions 302.

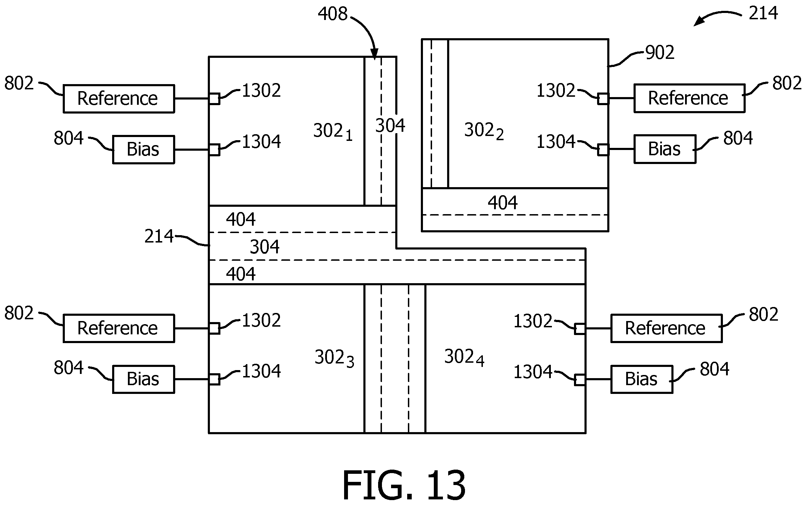

FIGURE 13 schematically illustrates a variation of FIGURE 8 in which the reference circuitry 802 and bias circuitry 804 are external to the processing regions 302. Each of the processing regions 302 includes electrical contacts 1302 and 1304 for the external the reference circuitry 802 and the bias circuitry 804. In another variation, at least one the reference circuitry 802 or bias circuitry 804 for a processing region 302 is internal to the processing region 302 (FIGURE 8) and at least one the reference circuitry 802 or bias circuitry 804 for a processing region 302 is external to the processing region 302 (FIGURE 13).

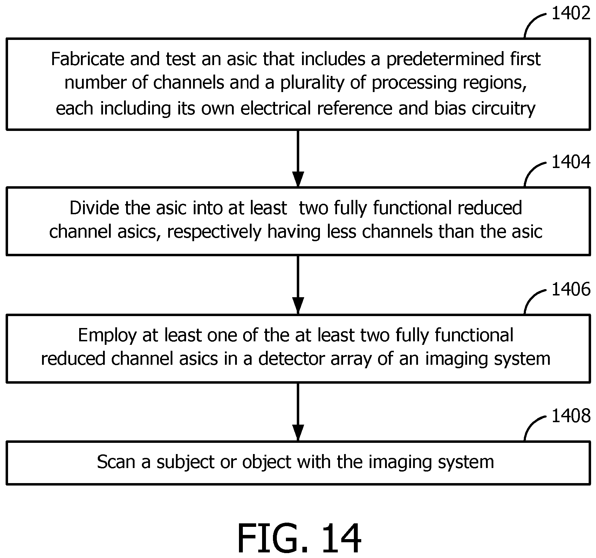

FIGURE 14 illustrates an example method in accordance with the description herein.

It is to be appreciated that the ordering of the acts in the methods described herein is not limiting. As such, other orderings are contemplated herein. In addition, one or more acts may be omitted and/or one or more additional acts may be included.

At 1402, an ASIC, including a predetermined first number of channels and a plurality of processing regions, each including its own electrical reference and bias circuitry, is fabricated and tested. As discussed herein, the ASIC is a single modular ASIC, with a single unitary die that is divisible into one or more reduced channel ASICs.

At 1404, the ASIC is divided into at least two fully functional reduced channel ASICS, respectively having a second and a third number of channels, wherein the second and the third number of channels is less that the first number of channels.

At 1406, the at least one of the at least two fully functional reduced channel ASICS are employed in a detector array of an imaging system.

At 1408, a subject or object is scanned with the imaging system.

The invention has been described with reference to the preferred embodiments.

## Claims

1. An imaging system detector array (112), comprising:
a detector tile (116), including:
a photosensor array (202), including a plurality of photosensor pixels (204)
a scintillator array (212) optically coupled to the photosensor array; and
an ASIC electronics layer (214) electrically coupled to the photosensor array, the electronics layer, including: a plurality of individual processing regions (302), **characterised by** each processing region including a predetermined number of channels corresponding to a sub-set of the plurality of photosensor pixels, wherein the processing regions are in electrical communication with each other, and each processing region includes its own electrical reference and bias circuitry (802, 804).

2. The imaging system detector array of claim 1, wherein the electronics layer is a sub-portion of a single unitary die (218) that includes a plurality of the electronics layers.

3. The imaging system detector array of claim 2, the electronics layer, further including:
a sub-portion of a digital electronics region shared by at least two adjacent electronics layers of the plurality of the electronics layers of the single unitary die.

4. The imaging system detector array of claim 3, wherein the digital electronics region resides between at least two of the processing regions.

5. The imaging system detector array of claim 4, the digital electronics region, comprising: a first sub-portion (1202₁) and a second sub-portion (1202₂), wherein the processing region resides between the first sub-portion and the second sub-portion of the digital electronics region.

6. The imaging system detector array of any of claims 2 to 5, the electronics layer, further including:
a sub-portion of an analog electronics region shared by at least two adjacent electronics layers of the plurality of the electronics layers of the single unitary die.

7. The imaging system detector array of any of claims 2 to 6, wherein the single die includes 512 channels and the electronics layer of the detector tile includes less than 512 channels.

8. The imaging system detector array of any of claims 2 to 6, wherein the electronics layer of the detector tile includes a first number of channels, wherein the first number of channels is from a group consisting of 16, 32, 64, 128, or 256 channels, and the single die includes a second number of channels, wherein the second number of channels is greater than the first number of channels.

9. The imaging system detector array of any of claims 1 to 6, wherein the electrical reference and bias circuitry of one of the processing regions is internal to the one of the processing regions.

10. The imaging system detector array of any of claims 1 to 9, wherein the electrical reference and bias circuitry of one of the processing regions is external to the one of the processing regions.

11. The imaging system detector array of any of claims 1 to 10, wherein the electronics layer is a sub-portion of a fabricated die having both functional and non-functional channels, wherein the electronics layer includes the functional channels and does not include the non-functional channels of the fabricated die.

12. The imaging system detector array of any of claims 1 to 11, the electronics layer, further comprising:
a current-to-frequency (I/F) converter that generates a train of pulses with a pulse frequency indicative of x-ray photons incident on a photosensor pixel.

13. The imaging system detector array of any of claims 1 to 12, the electronics layer, further comprising:
at least one of a flex material, a ceramic material, a silicon material, or a print circuit board.

14. A method, comprising:
fabricating and testing an ASIC that includes a first number of processing regions, each processing region including a predetermined number of channels and each processing region including its own electrical reference and bias circuitry;
dicing the ASIC into at least two fully functional reduced channel ASICS respectively having a second and a third number of channels, wherein the second and the third number of channels is less than the first number of channels; and
employing at least one of the at least two fully functional reduced channel ASICS in a detector array of an imaging system according to claim 1.

15. The method of claim 14, further comprising:
dicing the ASIC with one of a laser, a mechanical saw, or a scribe.

16. The method of any of claims 14 to 15, wherein the dicing removes an amount of a material of the ASIC, wherein the amount is in a range of 150 microns or less.

17. The method of any of claims 14 to 16, wherein the at least two fully functional reduced channel ASICS include at least one of a sub-portion of a digital electronics region shared by the at least two fully functional reduced channel ASICS prior to dicing or a sub-portion of a digital electronics region shared by the at least two fully functional reduced channel ASICS prior to dicing.

18. The method of any of claims 14 to 17, wherein the ASIC includes 512 channels and the at least two fully functional reduced channel ASICS include less than 512 channels.

19. The method of any of claims 14 to 17, wherein the at least two fully functional reduced channel ASICS respectively include a first number of channels and a second number of channels, and the ASIC includes a third number of channels, wherein the third number of channels is greater than the first and second number of channels.

20. An ASIC (218) for an imaging detector tile (116), comprising:
a plurality of individual processing regions (302), each processing region including a predetermined number of channels corresponding to a sub-set of a plurality of photosensor pixels of the detector tile, wherein the processing regions are in electrical communication with each other, and each processing region includes its own electrical reference and bias circuitry (802, 804).

21. The ASIC of claim 20, wherein each of the plurality of individual processing regions is a sub-portion of a single unitary die (218).

22. The ASIC of claim 21, further including:
a digital electronics region shared by at least two of the plurality of individual processing regions.

23. The ASIC of claim 21, further including:
an analog electronics region shared by at least two of the plurality of individual processing regions.

24. The ASIC of any of claims 21 to 23, wherein the single die includes 512 channels and the plurality of individual processing regions includes less than 512 channels.

25. The ASIC of any of claims 21 to 23, wherein the plurality of individual processing regions includes a first number of channels, wherein the first number of channels is from a group consisting of 16, 32, 64, 128, or 256 channels, and the single die includes a second number of channels, wherein the second number of channels is greater than the first number of channels.

26. The ASIC of any of claims 20 to 25, wherein the electrical reference and bias circuitry of one of the plurality of individual processing regions is internal to the one of the plurality of individual processing regions.

27. The ASIC of any of claims 20 to 25, wherein the electrical reference and bias circuitry of one of the plurality of individual processing regions is external to the one of the plurality of individual processing regions.

28. The ASIC of any of claims 20 to 27, wherein the plurality of individual processing regions is divisible into one or more fully functional reduced channel ASICs.

29. The ASIC of claim 28, wherein the plurality of individual processing regions are part of a first definition or first pixel count detector and is divisible into one or more second definition or second pixel count detectors, wherein the first definition is greater than the second definition or the first pixel count is greater than the second pixel count.

30. The ASIC of any of claims 20 to 29, further comprising: at least one feature with a size in a range of 0.05 micron to 0.20 micron.

31. The ASIC of any of claims 20 to 30, further comprising:
a current-to-frequency (I/F) converter that generates a train of pulses with a pulse frequency indicative of x-ray photons incident on a photosensor pixel.

## Patentansprüche

1. Bildgebungssystem-Detektorarray (112), umfassend:
eine Detektorkachel (116), umfassend:
ein Photosensorarray (202), umfassend eine Vielzahl von Photosensorpixeln (204);
ein Szintillatorarray (212), das optisch mit dem Photosensorarray gekoppelt ist;
eine ASIC-Elektronikschicht (214), die elektrisch mit dem Photosensorarray gekoppelt ist, wobei die Elektronikschicht Folgendes umfasst: eine Vielzahl von einzelnen Verarbeitungsregionen (302),
**dadurch gekennzeichnet, dass** jede Verarbeitungsregion eine vorgegebene Anzahl von Kanälen entsprechend einer Teilgruppe der Vielzahl von Photosensorpixeln umfasst, wobei die Verarbeitungsregionen in elektrischer Kommunikation miteinander stehen und jede Verarbeitungsregion ihre eigene elektrische Referenz- und Vorspannungsschaltung (802, 804) umfasst.

2. Bildgebungs-Detektorarray nach Anspruch 1, wobei die Elektronikschicht ein Teilabschnitt eines einzelnen einheitlichen Chips (218) ist, der eine Vielzahl der elektronischen Schichten umfasst.

3. Bildgebungs-Detektorarray nach Anspruch 2, wobei die Elektronikschicht weiterhin Folgendes umfasst:
einen Teilabschnitt einer Digitalelektronikregion, der durch mindestens zwei benachbarte Elektronikschichten der Vielzahl von Elektronikschichten des einzelnen einheitlichen Chips gemeinsam genutzt wird.

4. Bildgebungssystem-Detektorarray nach Anspruch 3, wobei sich die Digitalelektronikregion zwischen mindestens zwei der Verarbeitungsregionen befindet.

5. Bildgebungssystem-Detektorarray nach Anspruch 4, wobei die Digitalelektronikregion Folgendes umfasst: einen ersten Teilabschnitt (1202₁) und einen zweiten Teilabschnitt (1202₂), wobei sich die Verarbeitungsregion zwischen dem ersten Teilabschnitt und dem zweiten Teilabschnitt der Digitalelektronikregion befindet.

6. Bildgebungssystem-Detektorarray nach einem der Ansprüche 2 bis 5, wobei die Elektronikschicht weiterhin Folgendes umfasst:
einen Teilabschnitt einer Analogelektronikregion, der durch mindestens zwei benachbarte Elektronikschichten der Vielzahl von Elektronikschichten des einzelnen einheitlichen Chips gemeinsam genutzt wird.

7. Bildgebungssystem-Detektorarray nach einem der Ansprüche 2 bis 6, wobei der einzelne Chip 512 Kanäle umfasst und die Elektronikschicht der Detektorkachel weniger als 512 Kanäle umfasst.

8. Bildgebungssystem-Detektorarray nach einem der Ansprüche 2 bis 6, wobei die Elektronikschicht der Detektorkachel eine erste Anzahl von Kanälen umfasst, wobei die erste Anzahl von Kanälen aus einer Gruppe bestehend aus 16, 32, 64, 128 oder 256 Kanälen stammt und der einzelne Chip eine zweite Anzahl von Kanälen umfasst, wobei die zweite Anzahl von Kanälen größer als die erste Anzahl von Kanälen ist.

9. Bildgebungssystem-Detektorarray nach einem der Ansprüche 1 bis 6, wobei die elektrische Referenz- und Vorspannungsschaltung von einer der Verarbeitungsregionen innerhalb der einen von den Verarbeitungsregionen liegt.

10. Bildgebungssystem-Detektorarray nach einem der Ansprüche 1 bis 9, wobei die elektrische Referenz- und Vorspannungsschaltung von einer der Verarbeitungsregionen außerhalb der einen von den Verarbeitungsregionen liegt.

11. Bildgebungssystem-Detektorarray nach einem der Ansprüche 1 bis 10, wobei die Elektronikschicht ein Teilabschnitt von einem hergestellten Chip mit sowohl funktionalen als auch nicht-funktionalen Kanälen ist, wobei die Elektronikschicht die funktionalen Kanäle umfasst und die nicht-funktionalen Kanäle des hergestellten Chips nicht umfasst.

12. Bildgebungssystem-Detektorarray nach einem der Ansprüche 1 bis 11, wobei die Elektronikschicht weiterhin Folgendes umfasst:
einen Strom-Frequenz-(I/F) Wandler, der eine Impulsfolge mit einer Impulsfrequenz erzeugt, die auf ein Photosensorpixel auftreffende Röntgenphotonen angibt.

13. Bildgebungssystem-Detektorarray nach einem der Ansprüche 1 bis 12, wobei die Elektronikschicht weiterhin Folgendes umfasst:
mindestens ein flexibles Material, ein Keramikmaterial, ein Siliziummaterial oder eine gedruckte Leiterplatte.

14. Verfahren, umfassend:
Herstellen und Testen eines ASICs, das eine erste Anzahl von Verarbeitungsregionen umfasst, wobei jede Verarbeitungsregion eine vorgegebene Anzahl von Kanälen umfasst und jede Verarbeitungsregion ihre eigene elektrische Referenz- und Vorspannungsschaltung umfasst;
Vereinzeln des ASICs in mindestens zwei ASICs mit vollkommen funktionalen reduzierten Kanälen mit einer zweiten bzw. einer dritten Anzahl von Kanälen, wobei die zweite und die dritte Anzahl von Kanälen geringer ist als die erste Anzahl von Kanälen; und
Nutzen von mindestens einem der mindestens zwei ASICs mit vollkommen funktionalen reduzierten Kanälen in einem Detektorarray eines Bildgebungssystems nach Anspruch 1.

15. Verfahren nach Anspruch 14, weiterhin umfassend:
Vereinzeln des ASICs mit einem Laser, einer mechanischen Säge oder einem Ritzgerät.

16. Verfahren nach einem der Ansprüche 14 bis 15, wobei durch das Vereinzeln eine Materialmenge des ASICs entfernt wird, wobei die Menge in einem Bereich von 150 Mikron oder weniger liegt.

17. Verfahren nach einem der Ansprüche 14 bis 16, wobei die mindestens zwei ASICs mit vollständig funktionalen reduzierten Kanälen mindestens einen Teilabschnitt einer Digitalelektronikregion, der von den mindestens zwei ASICs mit vollständig funktionalen reduzierten Kanälen vor dem Vereinzeln gemeinsam genutzt wird, oder einen Teilabschnitt einer Digitalelektronikregion, der von den mindestens zwei ASICs mit vollständig funktionalen reduzierten Kanälen vor dem Vereinzeln gemeinsam genutzt wird, umfasst.

18. Verfahren nach einem der Ansprüche 14 bis 17, wobei das ASIC 512 Kanäle umfasst und die mindestens zwei ASICs mit vollständig funktionalen reduzierten Kanälen weniger als 512 Kanäle umfassen.

19. Verfahren nach einem der Ansprüche 14 bis 17, wobei die mindestens zwei ASICs mit vollständig funktionalen reduzierten Kanälen eine erste Anzahl von Kanälen bzw. eine zweite Anzahl von Kanälen umfassen, und das ASIC eine dritte Anzahl von Kanälen umfasst, wobei die dritte Anzahl von Kanälen größer ist als die erste und die zweite Anzahl von Kanälen.

20. ASIC (218) für eine Bildgebungsdetektorkachel (116), umfassend:
eine Vielzahl von einzelnen Verarbeitungsregionen (302), wobei jede Verarbeitungsregion eine vorgegebene Anzahl von Kanälen entsprechend einer Teilgruppe einer Vielzahl von Photosensorpixeln der Detektorkachel umfasst, wobei die Verarbeitungsregionen in elektrischer Kommunikation miteinander stehen und jede Verarbeitungsregion ihre eigene elektrische Referenz- und Vorspannungsschaltung (802, 804) umfasst.

21. ASIC nach Anspruch 20, wobei jede der Vielzahl von einzelnen Verarbeitungsregionen ein Teilabschnitt eines einzelnen einheitlichen Chips (218) ist.

22. ASIC nach Anspruch 21, weiterhin umfassend:
eine Digitalelektronikregion, die durch mindestens zwei der Vielzahl von einzelnen Verarbeitungsregionen gemeinsam genutzt wird.

23. ASIC nach Anspruch 21, weiterhin umfassend:
eine Analogelektronikregion, die durch mindestens zwei der Vielzahl von einzelnen Verarbeitungsregionen gemeinsam genutzt wird.

24. ASIC nach einem der Ansprüche 21 bis 23, wobei der einzelne Chip 512 Kanäle umfasst und die Vielzahl von einzelnen Verarbeitungsregionen weniger als 512 Kanäle umfasst.

25. ASIC nach einem der Ansprüche 21 bis 23, wobei die Vielzahl von einzelnen Verarbeitungsregionen eine erste Anzahl von Kanälen umfasst, wobei die erste Anzahl von Kanälen aus einer Gruppe bestehend aus 16, 32, 64, 128 oder 256 Kanäle stammt und der einzelne Chip eine zweite Anzahl von Kanälen umfasst, wobei die zweite Anzahl von Kanälen größer als die erste Anzahl von Kanälen ist.

26. ASIC nach einem der Ansprüche 20 bis 25, wobei die elektrische Referenz- und Vorspannungsschaltung von einer der Vielzahl von einzelnen Verarbeitungsregionen innerhalb der einen von der Vielzahl von einzelnen Verarbeitungsregionen liegt.

27. ASIC nach einem der Ansprüche 20 bis 25, wobei die elektrische Referenz- und Vorspannungsschaltung von einer der Vielzahl von einzelnen Verarbeitungsregionen außerhalb der einen von der Vielzahl von einzelnen Verarbeitungsregionen liegt.

28. ASIC nach einem der Ansprüche 20 bis 27, wobei die Vielzahl von einzelnen Verarbeitungsregionen in einen oder mehrere ASICs mit vollständig funktionalen reduzierten Kanälen unterteilbar ist.

29. ASIC nach Anspruch 28, wobei die Vielzahl von einzelnen Verarbeitungsregionen Teil eines ersten Definitions- oder ersten Pixelzählwertdetektors ist und in einen oder mehrere zweite Definitions- oder zweite Pixelzählwertdetektoren unterteilbar ist, wobei die erste Definition größer als die zweite Definition ist oder der erste Pixelzählwert größer als der zweite Pixelzählwert ist.

30. ASIC nach einem der Ansprüche 20 bis 29, weiterhin umfassend mindestens ein Merkmal mit einer Größe in einem Bereich von 0,05 Mikron bis 0,20 Mikron.

31. ASIC nach einem der Ansprüche 20 bis 30, weiterhin umfassend:
einen Strom-Frequenz-(I/F) Wandler, der eine Impulsfolge mit einer Impulsfrequenz erzeugt, die ein Photosensorpixel auftreffende Röntgenphotonen angibt.

## Revendications

1. Ensemble de détecteur pour système d'imagerie (112), comprenant :
un carreau de détecteur (116), incluant :
un ensemble de photocapteur (202), incluant une pluralité de pixels de photocapteur (204)
un ensemble de scintillateur (212) couplé optiquement à l'ensemble de photocapteur ; et
une couche électronique de circuit ASIC (214) couplée électriquement à l'ensemble de photocapteur, la couche électronique incluant : une pluralité de régions de traitement individuelles (302),
**caractérisé par** chaque région de traitement incluant un nombre prédéterminé de canaux correspondant à un sous-groupe de la pluralité de pixels de photocapteur, dans lequel les régions de traitement sont en communication électrique les unes avec les autres, et chaque région de traitement inclut sa propre référence électrique et son propre circuit de polarisation (802, 804).

2. Ensemble de détecteur pour système d'imagerie selon la revendication 1, dans lequel la couche électronique est une sous-partie d'une matrice unitaire unique (218) qui inclut une pluralité de couches électroniques.

3. Ensemble de détecteur pour système d'imagerie selon la revendication 2, la couche électronique incluant en outre :
une sous-partie d'une région électronique numérique partagée par au moins deux couches électroniques adjacentes de la pluralité de couches électroniques de la matrice unitaire unique.

4. Ensemble de détecteur pour système d'imagerie selon la revendication 3, dans lequel la région électronique numérique se situe entre au moins deux des régions de traitement.

5. Ensemble de détecteur pour système d'imagerie selon la revendication 4, la région électronique numérique comprenant : une première sous-partie (1202₁) et une deuxième sous-partie (1202₂), dans lequel la région de traitement se trouve entre la première sous-partie et la deuxième sous-partie de la région électronique numérique.

6. Ensemble de détecteur pour système d'imagerie selon l'une quelconque des revendications 2 à 5, la couche électronique incluant en outre :
une sous-partie d'une région électronique analogique partagée par au moins deux couches électroniques adjacentes de la pluralité des couches électroniques de la matrice unitaire unique.

7. Ensemble de détecteur pour système d'imagerie selon l'une quelconque des revendications 2 à 6, dans lequel la matrice unique inclut 512 canaux et la couche électronique du carreau de détecteur inclut moins de 512 canaux.

8. Ensemble de détecteur pour système d'imagerie selon l'une quelconque des revendications 2 à 6, dans lequel la couche électronique du carreau de détecteur inclut un premier nombre de canaux, dans lequel le premier nombre de canaux provient d'un groupe constitué de 16, 32, 64, 128 ou 256 canaux, et la matrice unique inclut un deuxième nombre de canaux, dans lequel le deuxième nombre de canaux est supérieur au premier nombre de canaux.

9. Ensemble de détecteur pour système d'imagerie selon l'une quelconque des revendications 1 à 6, dans lequel la référence électrique et le circuit de polarisation de l'une des régions de traitement sont internes à l'une des régions de traitement.

10. Ensemble de détecteur pour système d'imagerie selon l'une quelconque des revendications 1 à 9, dans lequel la référence électrique et le circuit de polarisation de l'une des régions de traitement sont externes à l'une des régions de traitement.

11. Ensemble de détecteur pour système d'imagerie selon l'une quelconque des revendications 1 à 10, dans lequel la couche électronique est une sous-partie d'une matrice fabriquée ayant des canaux à la fois fonctionnels et non fonctionnels, dans lequel la couche électronique inclut les canaux fonctionnels et n'inclut pas les canaux non fonctionnels de la matrice fabriquée.

12. Ensemble de détecteur pour système d'imagerie selon l'une quelconque des revendications 1 à 11, la couche électronique comprenant en outre :
un convertisseur de courant-fréquence (I/F) qui génère un train d'impulsions ayant une fréquence d'impulsion indicatrice des photons radiographiques incidents sur un pixel de photocapteur.

13. Ensemble de détecteur pour système d'imagerie selon l'une quelconque des revendications 1 à 12, la couche électronique comprenant en outre :
au moins l'un parmi un matériau souple, un matériau en céramique, un matériau en silicium ou une carte de circuit imprimé.

14. Procédé comprenant :
la fabrication et le test d'un circuit ASIC qui inclut un premier nombre de régions de traitement, chaque région de traitement incluant un nombre prédéterminé de canaux et chaque région de traitement incluant sa propre référence électrique et son propre circuit de polarisation ;
le découpage du circuit ASIC en au moins deux circuits ASIC à nombre de canaux réduit entièrement fonctionnels ayant respectivement un deuxième et un troisième nombre de canaux, dans lequel les deuxième et troisième nombres de canaux sont inférieurs au premier nombre de canaux ; et
l'utilisation d'au moins l'un des au moins deux circuits ASIC à nombre de canaux réduits entièrement fonctionnels dans un ensemble de détecteur d'un système d'imagerie selon la revendication 1.

15. Procédé selon la revendication 14, comprenant en outre :
le découpage du circuit ASIC avec l'un parmi un laser, une scie mécanique ou une gravure.

16. Procédé selon l'une quelconque des revendications 14 à 15, dans lequel le découpage retire une quantité de matériau du circuit ASIC, dans lequel la quantité se situe dans une plage de 150 microns ou inférieure.

17. Procédé selon l'une quelconque des revendications 14 à 16, dans lequel les au moins deux circuits ASIC à nombre de canaux réduits entièrement fonctionnels incluent au moins l'une parmi une sous-partie d'une région électronique numérique partagée par les au moins deux circuits ASIC à nombre de canaux réduit entièrement fonctionnels avant le découpage ou une sous-partie d'une région électronique numérique partagée par les au moins deux circuits ASIC à nombre de canaux réduit entièrement fonctionnels avant le découpage.

18. Procédé selon l'une quelconque des revendications 14 à 17, dans lequel le circuit ASIC inclut 512 canaux et les au moins deux circuits ASIC à nombre de canaux réduit entièrement fonctionnels incluent moins de 512 canaux.

19. Procédé selon l'une quelconque des revendications 14 à 17, dans lequel les au moins deux circuits ASIC à nombre de canaux réduit entièrement fonctionnels incluent respectivement un premier nombre de canaux et un deuxième nombre de canaux, et le circuit ASIC inclut un troisième nombre de canaux, dans lequel le troisième nombre de canaux est supérieur aux premier et deuxième nombres de canaux.

20. Circuit ASIC (218) pour un carreau de détecteur d'imagerie (116), comprenant :
une pluralité de régions de traitement individuelles (302), chaque région de traitement incluant un nombre prédéterminé de canaux correspondant à un sous-groupe d'une pluralité de pixels de photocapteurs du carreau de détecteur, dans lequel les régions de traitement sont en communication électrique les unes avec les autres, et chaque région de traitement inclut sa propre référence électrique et son propre circuit de polarisation (802, 804).

21. Circuit ASIC selon la revendication 20, dans lequel chacune de la pluralité des régions de traitement individuelles est une sous-partie d'une matrice unitaire unique (218).

22. Circuit ASIC selon la revendication 21, incluant en outre :
une région électronique numérique partagée par au moins deux de la pluralité des régions de traitement individuelles.

23. Circuit ASIC selon la revendication 21, incluant en outre :
une région électronique analogique partagée par au moins deux de la pluralité des régions de traitement individuelles.

24. Circuit ASIC selon l'une quelconque des revendications 21 à 23, dans lequel la matrice unique inclut 512 canaux et la pluralité des régions de traitement individuelles inclut moins de 512 canaux.

25. Circuit ASIC selon l'une quelconque des revendications 21 à 23, dans lequel la pluralité des régions de traitement individuelles inclut un premier nombre de canaux, dans lequel le premier nombre de canaux provient d'un groupe constitué de 16, 32, 64, 128 ou 256 canaux, et la matrice unique inclut un deuxième nombre de canaux, dans lequel le deuxième nombre de canaux est supérieur au premier nombre de canaux.

26. Circuit ASIC selon l'une quelconque des revendications 20 à 25, dans lequel la référence électrique et le circuit de polarisation de l'une de la pluralité des régions de traitement individuelles est interne à l'une de la pluralité des régions de traitement individuelles.

27. Circuit ASIC selon l'une quelconque des revendications 20 à 25, dans lequel la référence électrique et le circuit de polarisation de l'une de la pluralité des régions de traitement individuelles est externe à l'une de la pluralité des régions de traitement individuelles.

28. Circuit ASIC selon l'une quelconque des revendications 20 à 27, dans lequel la pluralité des régions de traitement individuelles est divisible en un ou plusieurs circuits ASIC à nombre de canaux réduit entièrement fonctionnels.

29. Circuit ASIC selon la revendication 28, dans lequel la pluralité des régions de traitement individuelles fait partie d'une première définition ou d'un premier détecteur de nombre de pixels et est divisible en une ou plusieurs deuxièmes définitions ou un ou plusieurs deuxièmes détecteurs de nombre de pixels, dans lequel la première définition est supérieure à la deuxième définition ou le premier nombre de pixels est supérieur au deuxième nombre de pixels.

30. Circuit ASIC selon l'une quelconque des revendications 20 à 29, comprenant en outre au moins une caractéristique ayant une taille dans une plage de 0,05 microns à 0,20 microns.

31. Circuit ASIC selon l'une quelconque des revendications 20 à 30, comprenant en outre :
un convertisseur de courant-fréquence (I/F) qui génère un train d'impulsions ayant une fréquence d'impulsion indicatrice des photons radiographiques incidents sur un pixel de photocapteur.
